# EUROPEAN PATENT SPECIFICATION

(11) **EP 3 868 428 B1**
(45) Date of publication and mention of the grant of the patent: **10.07.2024**
(21) Application number: 20305149.5
(22) Date of filing: 18.02.2020
(51) Int. Cl.: A61M 5/34, A61M 39/10

(54) **AN ADAPTOR FOR A MEDICAL CONTAINER AND A MEDICAL CONTAINER COMPRISING SAID ADAPTOR**
ADAPTER FÜR EINEN MEDIZINISCHEN BEHÄLTER UND MEDIZINISCHER BEHÄLTER MIT DIESEM ADAPTER
ADAPTATEUR POUR UN RÉCIPIENT MÉDICAL ET RÉCIPIENT MÉDICAL COMPORTANT UN TEL ADAPTATEUR

(43) Date of publication of application: 25.08.2021
(73) Proprietor: Becton Dickinson France, 38800 Le Pont-de-Claix (FR)
(72) Inventor: EUVRARD, Nicolas, Durham NC 27701 (US); RIVIER, Cédric, 38340 Voreppe (FR); DURAND, Lubin, 07170 Mirabel (FR); NICOLAS, Maxime, 38000 GRENOBLE (FR); JAMON, Rémi, 38410 VAULNAVEYS LE HAUT FRANCE (FR)
(74) Representative: Germain Maureau

(56) References cited:
- EP-A1- 3 381 503
- US-A1- 2006 033 331
- US-A1- 2013 076 030
- US-A1- 2014 339 811
- US-A1- 2015 119 863

## Description

The present invention relates to an adaptor for connecting a medical container to a connector and a medical container comprising said adaptor.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction of injection, with respect to an adaptor or a medical container of the invention, and the "proximal direction" is to be understood as meaning the opposite direction to said direction of injection, that is to say the direction towards the user's hand holding a medical container as for an injection operation.

Basically, the medical container usually comprise a container forming a reservoir for containing a medical product. The container has a distal end in the form of a longitudinal tip defining an axial passageway through which the medical product is expelled from the container. However, this longitudinal tip does not allow parenteral administration by itself and must either comprise a staked needle or an adaptor allowing the connection of the syringe to a connector such as a needle hub. This connector is typically screwed into the adaptor and engages the distal end of the container to establish a fluid path between the reservoir and said connector.

It is important that the connector be properly screwed into the adaptor. The end user has to apply a sufficient torque when screwing the connector into the adaptor in order to get a proper fitting of the connector onto the distal end of the container. Under-screwing into the adaptor may otherwise lead to an ejection of the connector or a leakage when transferring a fluid between the container and the connector, the leakage occuring between the connector and the tip of the syringe. Alternatively, over-screwing of the connector into the adaptor may lead to the rotation or tilt of the adaptor.

Document US2015/0119863 A1 describes an adaptor to improve a threaded connection between two or more devices, where a soft interface is provided between the devices. Documents US2014/0339811 A1, EP 3 381 503 A1 and US2006/033331 A1 relate to adaptors for connecting a medical container to a connector. Document US2013/0076030 A1 describes torque limiting fastening assemblies for use with fluidic couplings.

There is therefore a need for a medical container providing the end user with an indication that the connector is properly screwed into the adaptor and thus improving the end user confidence during a connection and an injection step.

An aspect of the invention is an adaptor for connecting a medical container to a connector having a proximal region provided with at least two outer wings at its proximal end, as defined in claim 1.

In particular, said predetermined rotation angle may correspond to a predetermined depth of the outer wings in the adaptor. Said predetermined depth is defined as being the depth above which there is no leaks. The leakage limit may be defined by the ISO 80369-7 of 2016, paragraphs 6.1 and 6.2. Said rotation angle may be about 180°, preferably said rotation angle is lower than 180°.

The adaptor of the invention thus permits the end user to know when the screwing of the connector into the adaptor should stop in order to avoid under- or over-screwing and therefore to avoid risks of leakage, ejection of the connector during a fluid transfer, or tilt or rotation of the adaptor. The indicator provides an increasing screwing torque until the outer wings overcome said predetermined rotation angle, after which the torque drops significantly. This provides the end user with a tactile indication that the connection is terminated. Thus, the outer wings reach a final position in the adaptor in which the connection between the connector and the adaptor is secured.

In embodiments, the indicator is further configured to produce a sound when the outer wings are further screwed beyond said predetermined rotation angle and/or when the outer wings suddenly deflect outwardly in the adaptor. The sound is preferably audible by a human ear. The indicator may therefore provide the end user with both a tactile and audible indication that the connection is terminated and secured. This therefore improves the end user confidence during the connection step and the injection step.

In embodiments, said engaging means comprise an internal thread located on an inner surface of said distal part of the adaptor. In such an embodiment, at least a portion of the indicator may be located in a root of said internal thread. The internal thread may be a double thread configured to enable said outer wings to reach said final position after a 180° rotation. This allows an easy and simple gesture for the end user, who is thereby allowed to perform a safe connection after 180° rotation only of the outer wings.

Alternatively or in combination, the internal thread may be a segmented thread. For example, the thread may discontinuously extend along a helical path on the inner surface of the distal part of the adaptor. The various segments of the thread that extend along the helical path may be positioned so that each outer wing always remains in contact with at least one segment of the thread. Two segments of the segmented thread may not be one above the other. Such a segmented thread allows molding the adaptor with two inserts.

The indicator comprises at least a resilient tongue, preferably two resilient tongues, capable of being at least partially circumferentially deflected by the outer wings at least in the screwing direction, preferably both in the screwing direction and in the unscrewing direction. Advantageously, the two resilient tongues are diametrically opposed. For example, the resilient tongue(s) may be radial and/or axial tongue(s). For example, during the connection, the resilient tongue(s) is/are capable of being deflected in the screwing direction when the outer wings exert a circumferential pressure on said tongue(s), said tongue(s) being capable of coming automatically back to a rest position once the outer wings have overcome the tongue(s). The deflection of the resilient tongue(s) necessitates that the end user applies an increasing torque for screwing the connector into the adaptor. In the same manner, during the disconnection of the connector from the adaptor, the resilient tongue(s) is/are capable of being deflected in the unscrewing direction when the outer wings exert a circumferential pressure on said tongue(s), said tongue(s) being capable of coming automatically back to a rest position once the outer wings have overcome the tongue(s). The deflection of the resilient tongue(s) necessitates that the end user applies an increasing torque for unscrewing the connector into the adaptor. For example, the resilient tongues need to be capable of being circumferentially deflected by the outer wings in the unscrewing direction when the adaptor is used for a drug reconstitution, i.e. when two different needles need to be successively connected to the adaptor.

In embodiments, the resilient tongue has an axial dimension equal to or lower than the screw pitch of the internal thread.

In embodiments, the indicator further comprises a straight stop configured to be contacted by the outer wings of the connector when said outer wings reach said final position in the adaptor. For example, the straight stop is a radial rib located at a proximal end of the internal thread.

The indicator further comprises a mechanical stop configured to be hit by the resilient tongue when the resilient tongue comes back to a rest configuration once overcome by said outer wings. When coming back to a rest configuration once overcome by the outer wings, the resilient tongue may go further than the rest configuration, hit the mechanical stop and after come back to the rest configuration. When the resilient tongue hits the mechanical stop, this may produce a sound indicating the end user that the connection of the connector into the adaptor is terminated and secured.

Another aspect of the invention is a medical container comprising a distal tip and the adaptor as described above, said adaptor being mounted onto the distal tip of the medical container.

Another aspect of the invention is a method for manufacturing the adaptor described above, said method comprising the step of forming the adaptor above by injection molding.

The invention and the advantages arising therefrom will clearly emerge from the detailed description that is given below with reference to the appended drawings as follows :
- Figure 1 is an exploded perspective view of the adaptor according to an embodiment of the invention, with the medical container intended to receive the adaptor and the connector intended to be screwed into the adaptor,
- Figures 2-5 are perspective views of the adaptor of Figure 1, taken from various angles showing the path of the internal thread and the indicator of the adaptor,
- Figures 6-9 are perspective views of an embodiment of the adaptor not part of the invention, taken from various angles showing the path of the internal thread and the indicator of the adaptor.

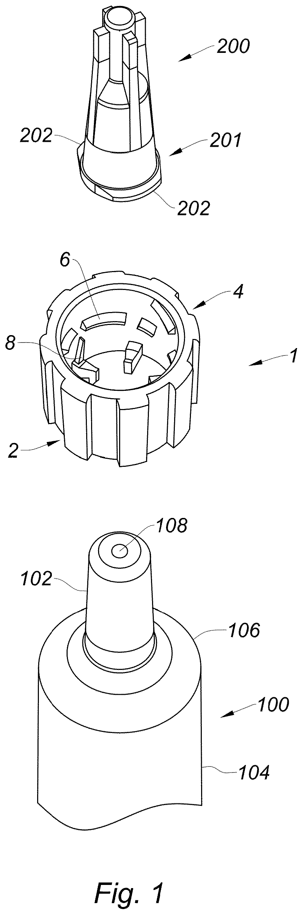

With reference to Figure 1 is shown an adaptor 1 according to an embodiment of the invention. The adaptor 1 is intended to be mounted onto the distal tip 102 of a medical container 100, in particular onto the outer surface of the distal tip 102. The outer surface of the distal tip 102 is generally distally tapered or frustoconical. Alternatively, it could be cylindrical. The adaptor 1 permits to connect a connector, such as a cap or a needle hub 200 having a proximal region 201 provided with at least two connecting means such as outer wings 202 at its proximal end, to the distal tip 102. As shown, the connector preferably comprises two outer wings 202 which are diametrically opposed.

With reference to Figures 1-5, the adaptor 1 comprises a proximal part 2, which may be in the form of a mounting ring, configured to be secured onto the distal tip 102 of the medical container 100, and a distal part 4, which may be in the form of a connecting ring, configured to receive the connector in order to establish a reliable fluid communication between a passageway 108 of the distal tip 102 and the connector.

The connector, such as the needle hub 200, is intended to be connected to the adaptor 1 via screwing.

In this view, the adaptor 1 has engaging means, such as an internal thread 6, configured to engage the outer wings 202 of the needle hub 200 in a screwing relationship. The outer wings 202 are intended to reach a final position in the adaptor 1, in which the connection between the needle hub 200 and the adaptor 1 is secured and a reliable fluid communication between the needle hub 200 and the distal tip 102 is ensured. Said final position is the leakage limit above which there is no leak between the medical container and the connector. The leakage limit may be defined by the ISO 80369-7 of 2016, paragraphs 6.1 and 6.2.

The adaptor 1 further comprises an indicator, such as a resilient tongue 8, configured to increase the resisting torque as the outer wings 202 are screwed into the adaptor 1 until they reach said final position corresponding to a predetermined rotation angle and to release said resisting torque when the outer wings 202 are further screwed beyond said predetermined rotation angle, the outer wings 202 thereby reaching a final position in the adaptor 1 in which the connection between the connector and the adaptor 1 is secured. The indicator therefore provides the end user with a tactile indication that the connection is terminated. As will appear below, the indicator may further be configured to produce a sound when the outer wings 202 reach their final position in the adaptor 1.

The internal thread 6 is located on the inner surface 4a of the distal part 4 of the adaptor 1. In the example shown, the internal thread 6 is a double thread (6a, 6b) with two symmetrical profiles : the double thread (6a, 6b) is dimensioned so as to allow the outer wings 202 to reach their final position in the adaptor 1 after a 180° rotation only. The end user may therefore perform the connection between the needle hub 200 and the adaptor 1 in a very quick and friendly gesture.

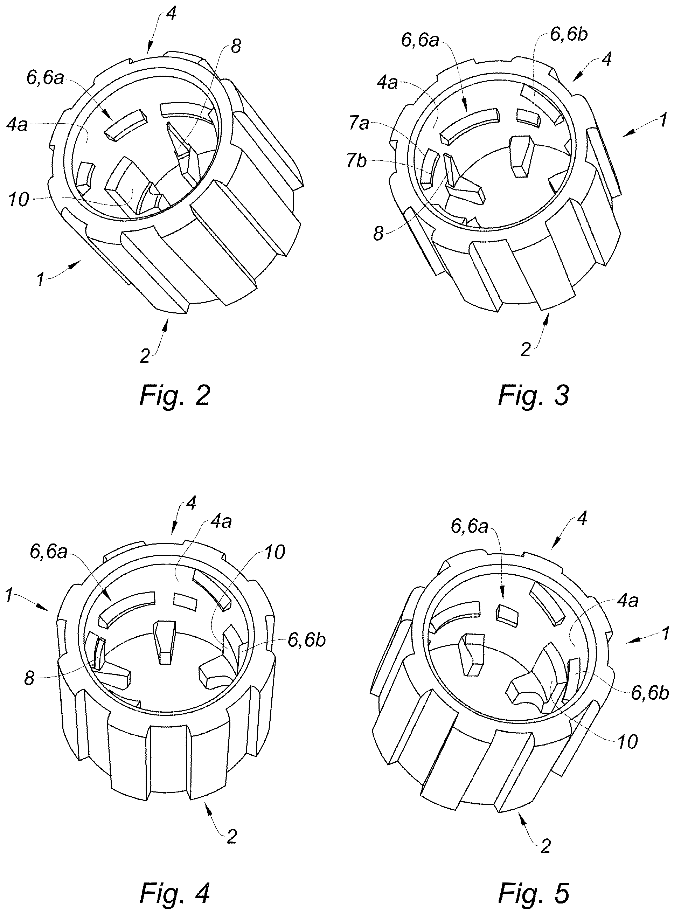

In the example shown, the double thread (6a, 6b) is further segmented. With reference to Figures 2-4, the double thread (6a, 6b) follows a helical path in a discontinuous way and is formed of several segments. The various segments of the double thread (6a, 6b) are positioned relative to one another so that each outer wing 202 remains in contact at least with one of the segments forming the thread (6a, 6b) during all the time it is screwed into the adaptor 1 or unscrewed from the adaptor 1. A segmented double thread implies the molding of the adaptor 1 with two inserts.

As appears from Figure 3, the double thread (6a, 6b) has a root 7a, which corresponds to the inner surface of the distal part 4, and a crest 7b, the cross section of the thread in the example shown being an isosceles triangle. In examples not shown, the cross section of the thread could be trapezoidal or semi-circular.

The resilient tongue 8 is located in the root 7a of the internal thread 6. Preferably, the resilient tongue 8 comprises a longitudinal surface which is flush with the inner surface 4a of the adaptor but not fixed to said inner surface 4a. A resilient tongue 8 may be present for each profile of the double thread (6a, 6b): the two resilient tongues 8 (only one being visible on Figures 2-5) are therefore diametrically opposed. The resilient tongue 8 has both an axial dimension and a radial dimension. Its axial dimension is preferably equal or lower, preferably strictly lower, than the screw pitch of the double thread (6a, 6b).

In the Figures 1-5, the resilient tongue 8 is shown in a rest position, in which it substantially extends in the axial direction. The resilient tongue 8 is capable of being deflected in the screwing direction and in the unscrewing direction under a circumferential pressure exerted by an outer wing 202 and to come back automatically to its rest position once said circumferential pressure is released.

The adaptor 1 further comprises a straight stop, which may be under the form of a radial rib 10 located at a proximal end of the internal thread 6. The radial rib 10 is present for each profile of the double thread (6a, 6b) : the two radial ribs 10 are therefore diametrically opposed. The straight stop is configured to be contacted by the outer wings 202 when the outer wings 202 reach their final position in the adaptor 1.

The operation of the adaptor 1 of Figures 1-5 is described herein below.

The user inserts the needle hub 200 into the distal end of the adaptor 1 and engages each of the two outer wings 202 of the needle hub 200 with one profile of the double thread (6a, 6b). The user starts screwing the needle hub 200 into the adaptor 1. As the user continues screwing the needle hub 200 into the adaptor 1, each outer wing 202 starts contacting the distal part of a resilient tongue 8. As a result, the resisting torque increases and the resilient tongue 8 deflects circumferentially in the screwing direction. As the user continues the screwing step, the resisting torque continues to increase until the outer wings 202 reach a predetermined rotation angle where they overcome the resilient tongues 8 which escape and come back to their rest position. The user can feel a drop in the resisting torque and the outer wings 202 reach their final position in the adaptor 1, where the connection between the needle hub 200 and the adaptor 1 is secured. The drop in the resisting torque and optionally the sound produced by the resilient tongues 8 hitting a mechanical stop (not shown) therefore provide the end user with both a tactile indication and an audible indication that the connection between the needle hub 200 and the adaptor 1 is terminated and secured. The mechanical stop may be configured to be hit by the resilient tongue 8 when the resilient tongue 8 comes back to a rest configuration after escaping the outer wing, and further configured to produce a sound audible by the end user when being hit by said resilient tongue 8. The end user may therefore feel confident that under- or over-screwing has been avoided and that risks of leakage or of an ejection of the connector during a fluid transfer are avoided.

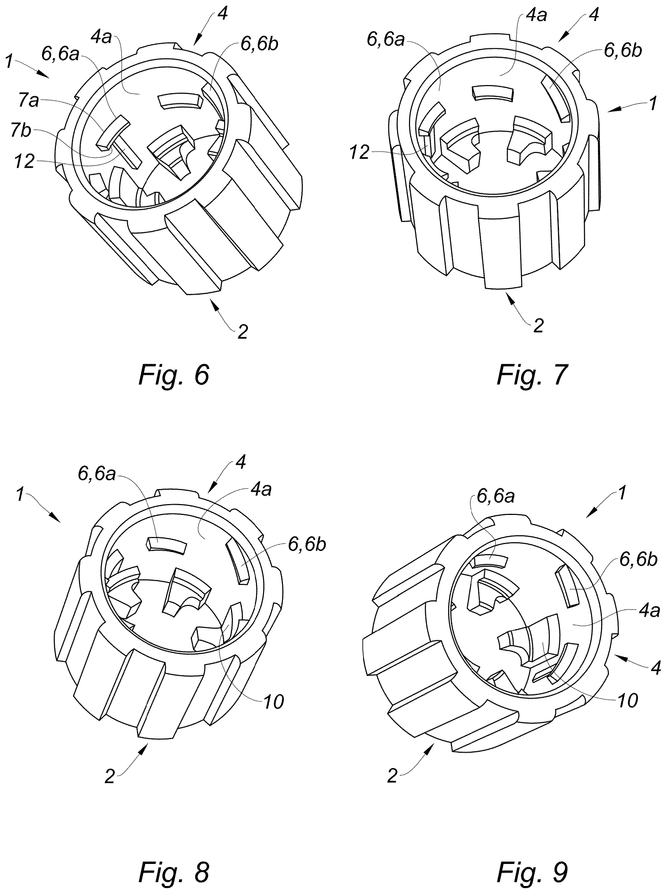

With reference to Figures 6-9, an embodiment of an adaptor 1 not part of the invention will be described, in which the resilient tongue is replaced by a bump 12. The references designating the same elements as in Figures 1-5 have been maintained.

The adaptor 1 of Figures 6-9 is provided with the same double thread (6a, 6b) as the adaptor of Figures 1-5. The bump 12 extends axially in the root 7a of the double thread (6a, 6b) for each profile of said thread. The two bumps 12 (only one being visible on the figures) are therefore diametrically opposed. The axial dimension of the bump 12 is preferably equal or lower than the screw pitch of the double thread (6a, 6b)..

The operation of the adaptor 1 of Figures 6-9 is described herein below.

The user inserts the needle hub 200 into the distal end of the adaptor 1 and engages each of the two outer wings 202 of the needle hub 200 with one profile of the double thread (6a, 6b). The user starts screwing the needle hub 200 into the adaptor 1. As the user continues screwing the needle hub 200 into the adaptor 1, each outer wing 202 starts contacting the distal part of a bump 12. As a result, the resisting torque increases and each outer wing 202 is caused to deform inwardly radially. As the user continues the screwing step, the resisting torque continues to increase until the outer wings 202 reach a predetermined rotation angle where they overcome the bumps 12 and where they come back to their undeformed configuration. The user can feel a drop in the resisting torque and the outer wings 202 reach their final position in the adaptor 1, where the connection between the needle hub 200 and the adaptor 1 is secured.

While coming back to its undeformed configuration, each outer wing 202 has hit the inner surface 4a of the distal part 4 with a force sufficient to produce a sound audible by the end user. In addition, while reaching their final position, each outer wing 202 may also have contacted the radial rib 10 located at the proximal end of the profile of the double thread (6a, 6b) with a force sufficient for producing a sound audible by the end user. The drop in the resisting torque and the sound produced by the outer wings 202 hitting the inner surface 4a of the distal part 4 and/or the radial ribs 10 therefore provide the end user with a tactile indication and an audible indication that the connection between the needle hub 200 and the adaptor 1 is terminated and correct. The end user may therefore feel confident that under- or over-screwing have been avoided and that risks of leakage or of an ejection of the connector during a fluid transfer are avoided.

The adaptor 1 may be made of a plastic material, more precisely of any rigid polymer adapted to medical use, such as high density polyethylene (PE), polypropylene (PP), polycarbonate (PC), acrylonitrile butadiene styrene (ABS), polyoxymethylene (POM), polystyrene (PS), polybutylene terephthalate (PBT), polyamide (PA), and combinations thereof. Preferably, the adaptor 1 is made of polycarbonate (PC).

The adaptor 1 may be manufactured by injection molding, for example with two inserts.

With reference to Figure 1, the invention also relates to a medical container 100 comprising a distal tip 102 and an adaptor 1 having the above-described features, said adaptor 1 being mounted onto the distal tip 102 of the medical container 100. The medical container 100, which may be a syringe, such as pre-fillable or prefilled syringe, comprises a tubular barrel 104 that defines a reservoir for a medical product. The tubular barrel 104 is provided with the distal tip 102 that may protrude from a distal shoulder 106. The distal tip 102 may be cylindrical or distally tapered. The distal tip 102 includes an internal passageway 108 in communication with the reservoir. The tubular barrel 104 and the distal tip 102 may be made of glass or of plastic material. The medical container 100 may be provided with a piston and a plunger rod (not shown) so as to expel the medical product from the reservoir and through the passageway 108. The adaptor 1 may be secured to the distal tip 102 by gluing, screwing, friction force or snap fitting.

The adaptor of the invention allows therefore improving the end user confidence during connection and injection.

## Claims

1. Adaptor (1) for connecting a medical container (100) to a connector (200) having a proximal region provided with at least two outer wings (202) at its proximal end, the adaptor (1) comprising :
a proximal part (2) configured to be secured onto a distal tip of a medical container (100),
a distal part (4) configured to receive said proximal region of said connector (200) in order to establish a fluid communication between the distal tip (102) of the medical container (100) and the connector (200),
engaging means (6, 6a, 6b) configured to engage said outer wings (202) of said connector (200) in a screwing relationship,
an indicator (8; 12) configured to increase the resisting torque as the outer wings (202) are screwed into the adaptor (1) until they reach a predetermined rotation angle and to release said resisting torque when the outer wings (202) are further screwed beyond said predetermined rotation angle, thereby providing an end user with a tactile indication that the connection between the connector and the adaptor is terminated, said indicator comprising at least one resilient tongue (8) capable of being circumferentially deflected by the outer wings (202) at least in the screwing direction,
**characterized in that**
the indicator further comprises a mechanical stop configured to be hit by said resilient tongue (8) when said resilient tongue (8) comes back to a rest configuration once overcome by said outer wings (202), the mechanical stop being further configured to produce a sound audible by the end user when hit by said resilient tongue (8).

2. The adaptor (1) according to claim 1, wherein the indicator (8; 12) is further configured to produce a sound when the outer wings (202) are further screwed beyond said predetermined rotation angle.

3. The adaptor (1) according to claim 1 or 2, wherein said engaging means comprise an internal thread (6, 6a, 6b) located on an inner surface (4a) of said distal part (4) of the adaptor (1).

4. The adaptor (1) according to the preceding claim, wherein at least a portion of the indicator (8; 12) is located in a root (7a) of said internal thread (6, 6a, 6b).

5. The adaptor (1) according to claim 3 or 4, wherein the internal thread is a segmented thread (6, 6a, 6b).

6. The adaptor (1) according to any of claims 1 to 5, wherein the indicator comprises two resilient tongues (8), capable of being circumferentially deflected by the outer wings (202) at least in the screwing direction, preferably both in the screwing direction and in the unscrewing direction.

7. The adaptor (1) according to claim 6, wherein said resilient tongue (8) has an axial dimension equal to or lower than the screw pitch of the internal thread (6, 6a, 6b).

8. The adaptor (1) according to any of the preceding claims, wherein the indicator further comprises a straight stop (10) configured to be contacted by the outer wings (202) when said outer wings (202) reach said final position in the adaptor (1).

9. The adaptor (1) according to claim 8, wherein the straight stop is a radial rib (10) located at a proximal end of said internal thread (6, 6a, 6b).

10. Medical container (100) comprising a distal tip (102) and the adaptor (1) according to any of the preceding claims, said adaptor (1) being mounted onto the distal tip (102) of the medical container (100).

11. A method for manufacturing the adaptor (1) according to any one of claims 1-9, said method comprising the step of forming the adaptor by injection molding.

## Patentansprüche

1. Adapter (1) zum Verbinden eines medizinischen Behälters (100) mit einem Verbindungsstück (200), das einen proximalen Bereich aufweist, der an seinem proximalen Ende mit mindestens zwei äußeren Flügeln (202) versehen ist, wobei der Adapter (1) Folgendes umfasst:
einen proximalen Teil (2), der dazu konfiguriert ist, an einer distalen Spitze eines medizinischen Behälters (100) befestigt zu werden,
einen distalen Teil (4), der dazu konfiguriert ist, den proximalen Bereich des Verbindungsstücks (200) aufzunehmen, um eine Fluidverbindung zwischen der distalen Spitze (102) des medizinischen Behälters (100) und dem Verbindungsstück (200) herzustellen,
Eingriffsmittel (6, 6a, 6b), die dazu konfiguriert sind, die äußeren Flügel (202) des Verbindungsstücks (200) in einer Schraubbeziehung in Eingriff zu nehmen,
ein Indikator (8; 12), der dazu konfiguriert ist, das Widerstandsmoment zu erhöhen, wenn die äußeren Flügel (202) in den Adapter (1) eingeschraubt werden, bis sie einen vorbestimmten Drehwinkel erreichen, und das Widerstandsmoment zu lösen, wenn die äußeren Flügel (202) weiter über den vorbestimmten Drehwinkel hinaus eingeschraubt werden, wodurch einem Endbenutzer ein haptischer Hinweis bereitgestellt wird, dass die Verbindung zwischen dem Verbindungsstück und dem Adapter beendet ist, wobei der Indikator mindestens eine elastische Zunge (8) umfasst, die durch die äußeren Flügel (202) in Umfangsrichtung mindestens in der Schraubrichtung ausgelenkt werden kann,
**dadurch gekennzeichnet, dass**
der Indikator ferner einen mechanischen Anschlag umfasst, der dazu konfiguriert ist, von der elastischen Zunge (8) getroffen zu werden, wenn die elastische Zunge (8), nachdem sie die äußeren Flügel (202) überwunden hat, in eine Ruhekonfiguration zurückkehrt, wobei der mechanische Anschlag ferner dazu konfiguriert ist, ein hörbares Geräusch durch den Endbenutzer zu erzeugen, wenn er von der elastischen Zunge (8) getroffen wird.

2. Adapter (1) nach Anspruch 1, wobei der Indikator (8; 12) ferner dazu konfiguriert ist, ein Geräusch zu erzeugen, wenn die äußeren Flügel (202) weiter über den vorbestimmten Drehwinkel hinaus eingeschraubt werden.

3. Adapter (1) nach Anspruch 1 oder 2, wobei die Eingriffsmittel ein Innengewinde (6, 6a, 6b) umfassen, das sich auf einer Innenfläche (4a) des distalen Teils (4) des Adapters (1) befindet.

4. Adapter (1) nach dem vorhergehenden Anspruch, wobei sich mindestens ein Abschnitt des Indikators (8; 12) in einem Gewindegrund (7a) des Innengewindes (6, 6a, 6b) befindet.

5. Adapter (1) nach Anspruch 3 oder 4, wobei das Innengewinde ein segmentiertes Gewinde (6, 6a, 6b) ist.

6. Adapter (1) nach einem der Ansprüche 1 bis 5, wobei der Indikator zwei elastische Zungen (8) umfasst, die durch die äußeren Flügel (202) in Umfangsrichtung mindestens in der Schraubrichtung, vorzugsweise sowohl in der Schraubrichtung als auch in der Löserichtung, ausgelenkt werden können.

7. Adapter (1) nach Anspruch 6, wobei die elastische Zunge (8) eine axiale Abmessung aufweist, die gleich oder kleiner als die Gewindesteigung des Innengewindes (6, 6a, 6b) ist.

8. Adapter (1) nach einem der vorhergehenden Ansprüche, wobei der Indikator ferner einen geraden Anschlag (10) umfasst, der dazu konfiguriert ist, von den äußeren Flügeln (202) berührt zu werden, wenn die äußeren Flügel (202) die Endposition im Adapter (1) erreichen.

9. Adapter (1) nach Anspruch 8, wobei der gerade Anschlag eine radiale Rippe (10) ist, die sich an einem proximalen Ende des Innengewindes (6, 6a, 6b) befindet.

10. Medizinischer Behälter (100), der eine distale Spitze (102) und den Adapter (1) nach einem der vorhergehenden Ansprüche umfasst, wobei der Adapter (1) auf der distalen Spitze (102) des medizinischen Behälters (100) montiert ist.

11. Verfahren zum Herstellen des Adapters (1) nach einem der Ansprüche 1-9, wobei das Verfahren den Schritt des Bildens des Adapters durch Spritzgießen umfasst.

## Revendications

1. Adaptateur (1) pour relier un récipient médical (100) à un raccord (200) ayant une région proximale munie d'au moins deux ailettes externes (202) au niveau de son extrémité proximale, l'adaptateur (1) comprenant :
une partie proximale (2) configurée pour être fixée sur une pointe distale d'un récipient médical (100),
une partie distale (4) configurée pour recevoir ladite région proximale dudit raccord (200) afin d'établir une communication fluidique entre la pointe distale (102) du récipient médical (100) et le raccord (200),
des moyens d'engagement (6 , 6a, 6b) configurés pour s'engager avec lesdites ailettes externes (202) dudit raccord (200) dans une relation de vissage,
un indicateur (8 ; 12) configuré pour augmenter le couple résistant à mesure que les ailettes externes (202) sont vissées dans l'adaptateur (1) jusqu'à ce qu'elles atteignent un angle de rotation prédéterminé et pour libérer ledit couple résistant lorsque les ailettes externes (202) sont davantage vissées au-delà dudit angle de rotation prédéterminé, fournissant ainsi à un utilisateur final une indication tactile selon laquelle la liaison entre le raccord et l'adaptateur est terminée, ledit indicateur comprenant au moins une languette élastique (8) capable d'être déviée circonférentiellement par les ailettes externes (202) au moins dans la direction de vissage,
**caractérisé en ce que**
l'indicateur comprend en outre une butée mécanique configurée pour être heurtée par ladite languette élastique (8) lorsque ladite languette élastique (8) revient à une configuration de repos une fois surmontée par lesdites ailettes externes (202), la butée mécanique étant en outre configurée pour produire un son audible par l'utilisateur final lorsqu'elle est heurtée par ladite languette élastique (8).

2. Adaptateur (1) selon la revendication 1, dans lequel l'indicateur (8 ; 12) est en outre configuré pour produire un son lorsque les ailettes externes (202) sont davantage vissées au-delà dudit angle de rotation prédéterminé.

3. Adaptateur (1) selon la revendication 1 ou 2, dans lequel lesdits moyens d'engagement comprennent un filetage interne (6, 6a, 6b) situé sur une surface interne (4a) de ladite partie distale (4) de l'adaptateur (1).

4. Adaptateur (1) selon la revendication précédente, dans lequel au moins une partie de l'indicateur (8 ; 12) est située dans un fond (7a) dudit filetage interne (6, 6a, 6b).

5. Adaptateur (1) selon la revendication 3 ou 4, dans lequel le filetage interne est un filetage segmenté (6, 6a, 6b).

6. Adaptateur (1) selon l'une des revendications 1 à 5, dans lequel l'indicateur comprend deux languettes élastiques (8), capables d'être déviées circonférentiellement par les ailettes externes (202) au moins dans la direction de vissage, de préférence à la fois dans la direction de vissage et dans la direction de dévissage.

7. Adaptateur (1) selon la revendication 6, dans lequel ladite languette élastique (8) a une dimension axiale inférieure ou égale au pas de vis du filetage interne (6, 6a, 6b).

8. Adaptateur (1) selon l'une des revendications précédentes, dans lequel l'indicateur comprend en outre une butée droite (10) configurée pour être en contact avec les ailettes externes (202) lorsque lesdites ailettes externes (202) atteignent ladite position finale dans l'adaptateur (1).

9. Adaptateur (1) selon la revendication 8, dans lequel la butée droite est une nervure radiale (10) située au niveau d'une extrémité proximale dudit filetage interne (6, 6a, 6b).

10. Récipient médical (100) comprenant une pointe distale (102) et l'adaptateur (1) selon l'une des revendications précédentes, ledit adaptateur (1) étant monté sur la pointe distale (102) du récipient médical (100).

11. Procédé de fabrication de l'adaptateur (1) selon l'une quelconque des revendications 1 à 9, ledit procédé comprenant l'étape de formation de l'adaptateur par moulage par injection.
